(19) Europäisches Patentamt
European Patent Office
Office européen des brevets

(11) **EP 4 714 539 A1**

(12) **EUROPEAN PATENT APPLICATION**
published in accordance with Art. 153(4) EPC

(43) Date of publication:
**25.03.2026 Bulletin 2026/13**

(21) Application number: **24806939.5**

(22) Date of filing: **16.04.2024**

(51) International Patent Classification (IPC):
***B01J 20/22*** *(2006.01)* ***B01D 53/14*** *(2006.01)*
***B01D 53/62*** *(2006.01)* ***B01D 53/81*** *(2006.01)*
***B01J 20/24*** *(2006.01)* ***B01J 20/26*** *(2006.01)*
***B01J 20/28*** *(2006.01)* ***B01J 20/34*** *(2006.01)*
***C01B 32/50*** *(2017.01)* ***C07B 61/00*** *(2006.01)*
***C07C 211/14*** *(2006.01)*

(52) Cooperative Patent Classification (CPC):
Y02C 20/40

(86) International application number:
**PCT/JP2024/015054**

(87) International publication number:
**WO 2024/236985 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.05.2023 JP 2023080538**
**01.12.2023 JP 2023204252**

(71) Applicant: **MITSUBISHI GAS CHEMICAL COMPANY, INC.**
**Chiyoda-ku**
**Tokyo 100-8324 (JP)**

(72) Inventors:
- **MAKINOSHIMA, Takashi**
  **Hiratsuka-shi, Kanagawa 254-0016 (JP)**
- **YONEHAMA, Shinichi**
  **Hiratsuka-shi, Kanagawa 254-0016 (JP)**

(74) Representative: **Hoffmann Eitle**
**Patent- und Rechtsanwälte PartmbB**
**Arabellastraße 30**
**81925 München (DE)**

(54) **CARBON DIOXIDE ABSORBENT, METHOD FOR RECOVERING CARBON DIOXIDE, AND APPARATUS FOR SEPARATING AND RECOVERING CARBON DIOXIDE**

(57) Provided are a carbon dioxide absorbent comprising an amine compound (A) represented by the following general formula (1) and a porous material (B), a method for capturing carbon dioxide using the carbon dioxide absorbent, and a carbon dioxide separation and capture apparatus. In the formula (1), each $R^1$ independently represents a hydroxy group or an organic group having 1 to 10 carbon atoms. n1 to n4 each independently represent a number of 1 to 8, and m represents a number of 0 to 10.

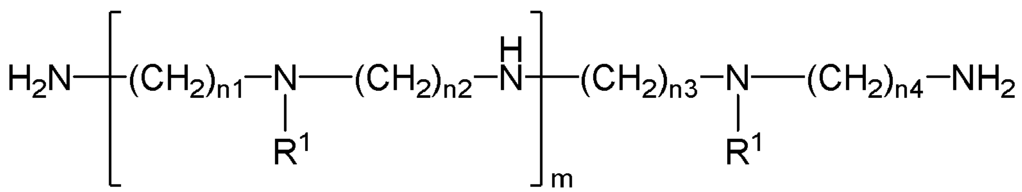
(1)

EP 4 714 539 A1

[Fig. 1]

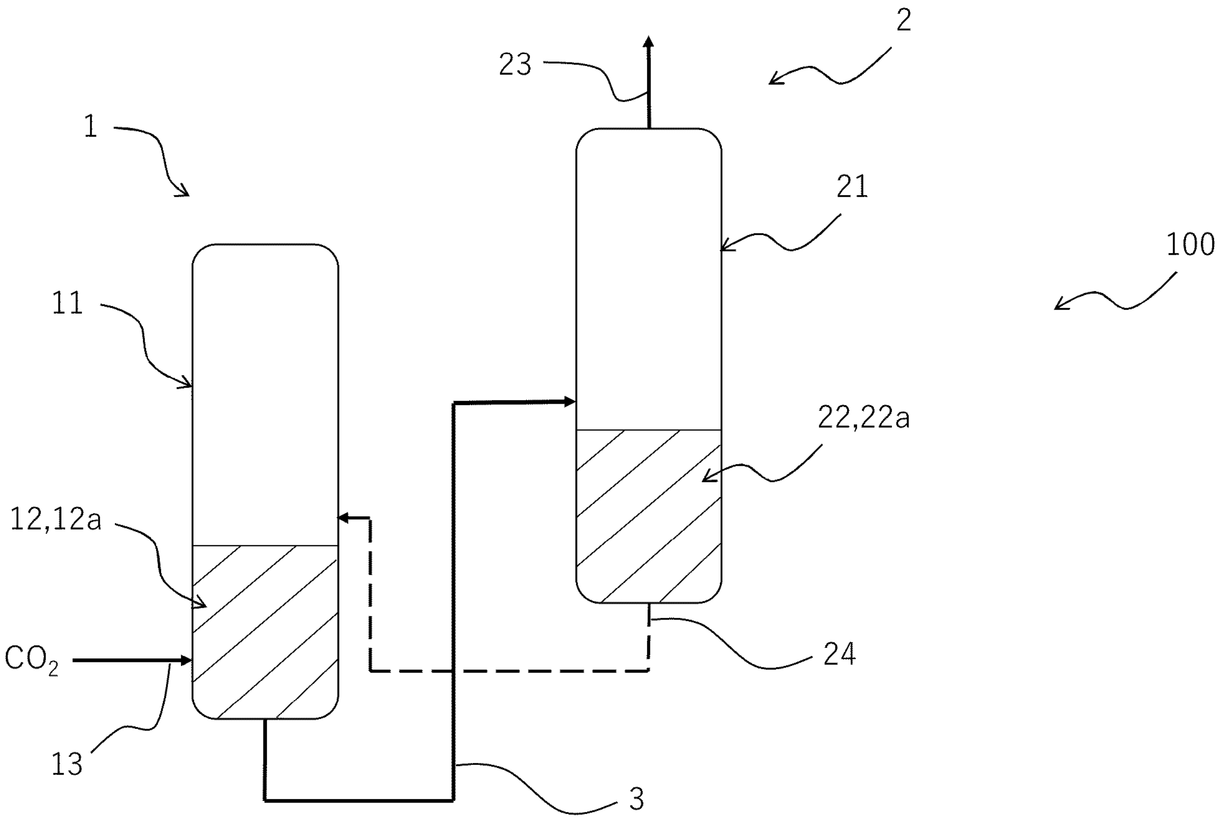

## Description

### Technical Field

The present invention relates to a carbon dioxide absorbent, a method for capturing carbon dioxide, and a carbon dioxide separation and capture apparatus.

### Background Art

From a viewpoint of global warming issues, there is a need to reduce carbon dioxide.

One of methods for reducing carbon dioxide is a method for capturing carbon dioxide using the carbon dioxide absorbent. As the carbon dioxide absorbent, aqueous solutions of amine compounds such as monoethanolamine are generally used. The aqueous solutions of the amine compounds have a property that they do not release carbon dioxide absorbed if not a high temperature of, e.g., 120°C or more, and if a carbon dioxide release temperature is set above the boiling point of water, a lot of energy is required to capture carbon dioxide because of high latent and specific heat of water.

As described above, one issue with respect to conventional carbon dioxide absorbents is further energy conservation when separating and capturing carbon dioxide. Another issue with respect to the conventional carbon dioxide absorbents is to reduce volatility of the amine compounds contained in the carbon dioxide absorbents, because a small amount of the amine compounds is volatilized and lost when gas is brought into contact with them in a step of absorbing carbon dioxide.

In addition, in a conventional chemical absorption method, the carbon dioxide absorbents are regenerated by boiling them with steam heating until a temperature of around 110°C to 130°C. Therefore, this method requires a very large amount of thermal energy. Furthermore, the thermal stability of the carbon dioxide absorbent is also an issue because there is concern that the amine compounds contained in the carbon dioxide absorbents may thermally decompose during this regeneration step.

Recently, the carbon dioxide absorbent having the amine compound supported on a porous material has been studied. Solidifying the amine compound by supporting it on the porous material enables carbon dioxide to be captured at lower energy than those of the aqueous solutions, of which the high latent and specific heats are problems.

Technologies related to such solidified carbon dioxide absorbents includes, for example, those described in Patent Literatures 1 to 3.

Patent Literature 1 discloses a solid absorbent for separating and capturing carbon dioxide which contains a specific alkanolamine and in which the alkanolamine is supported on a support.

Patent Literature 2 discloses a carbon dioxide absorbent, in which an amine compound is supported onto porous particles in which hydrophilic fibers and porous powder are composited by a hydrophilic binder.

Patent Literature 3 discloses a carbon dioxide absorbent composition containing polyethylene polyamine, phosphoric acid and/or phosphate, and silica.

### Citation List

#### Patent Literature

PTL1: JP 2012-139622 A
PTL2: JP 2018-187574 A
PTL3: JP 2020-58966 A

### Summary of Invention

#### Technical Problem

According to studies of the present inventors, however, carbon dioxide absorbents containing an amine compound and a porous material, such as those described in Patent Literatures 1 to 3, have room for improvement in terms of repeated usability.

The present invention has been made in view of the above circumstances, and an object of the present invention is to provide a carbon dioxide absorbent with a good carbon dioxide absorption property and particularly excellent repeated usability, a method for capturing carbon dioxide using the carbon dioxide absorbent, and a carbon dioxide separation and capture apparatus.

Solution to Problem

The present inventors have found that a carbon dioxide absorbent containing an amine compound having a specific structure and a porous material can attain the above object.

That is, the present invention relates to the following.

[1] A carbon dioxide absorbent comprising an amine compound (A) represented by the following general formula (1) and a porous material (B):

[Formula 1]

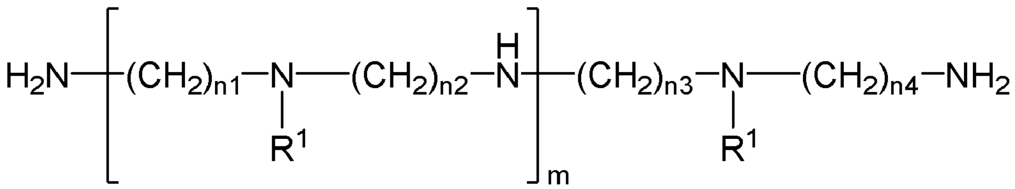

(1)

wherein in the formula (1), each $R^1$ independently represents a hydroxy group or an organic group having 1 to 10 carbon atoms; n1 to n4 each independently represent a number of 1 to 8; and m represents a number of 0 to 10.

[2] The carbon dioxide absorbent according to [1], wherein in the general formula (1), n1 to n4 are each independently a number of 3 to 8, and m is a number of 1 to 10.

[3] The carbon dioxide absorbent according to [1] or [2], wherein in the general formula (1), all of n1 to n4 are 3.

[4] The carbon dioxide absorbent according to any one of [1] to [3], wherein at least some of the amine compound (A) is supported on the porous material (B).

[5] The carbon dioxide absorbent according to any one of [1] to [4], wherein the porous material (B) comprises at least one selected from the group consisting of porous silica and porous alumina.

[6] The carbon dioxide absorbent according to any one of [1] to [5], wherein the porous material (B) is in a particulate form.

[7] The carbon dioxide absorbent according to [6], wherein a volume median particle size ($D_{50}$) of the porous material (B) as measured by laser diffraction/scattering particle size distribution measurement is 1 μm or more and 500 μm or less.

[8] The carbon dioxide absorbent according to any one of [1] to [7], wherein a specific surface area of the porous material (B) as measured by a BET method is 2 $m^2/g$ or more and 3,000 $m^2/g$ or less.

[9] The carbon dioxide absorbent according to any one of [1] to [8], wherein a pore size of the porous material (B) is 5 nm or more.

[10] The carbon dioxide absorbent according to any one of [1] to [9], wherein a pore volume of the porous material (B) is 0.1 $cm^3/g$ or more and 5.0 $cm^3/g$ or less.

[11] The carbon dioxide absorbent according to any one of [1] to [10], wherein a content of the amine compound (A) in the carbon dioxide absorbent is 0.1 parts by mass or more and 1,000 parts by mass or less with respect to 100 parts by mass of the porous material (B).

[12] The carbon dioxide absorbent according to any one of [4] to [11], wherein a burial rate for pores of the carbon dioxide absorbent calculated according to the following expression is 90% or less:

Burial rate for pores (%) of the carbon dioxide absorbent = {Pore volume ($cm^3/g$) of the porous material (B) - Pore volume ($cm^3/g$) of the carbon dioxide absorbent} / Pore volume ($cm^3/g$) of the porous material (B) $\times$ 100.

[13] A method for capturing carbon dioxide, comprising using the carbon dioxide absorbent according to any one of [1] to [12].

[14] The method according to [13], wherein the method comprises:

an absorption step of bringing a gas containing carbon dioxide into contact with the carbon dioxide absorbent, to cause the carbon dioxide absorbent to absorb the carbon dioxide, and

a desorption step of desorbing carbon dioxide from the carbon dioxide absorbent with carbon dioxide absorbed in the absorption step, and

wherein the desorption step comprises at least one step selected from the group consisting of the following (I) to (III):

(I) a step of subjecting the carbon dioxide absorbent with carbon dioxide absorbed to a reduced pressure condition;

(II) a step of bringing an inert gas not containing carbon dioxide into contact with the carbon dioxide absorbent with carbon dioxide absorbed; and

(III) a step of heating the carbon dioxide absorbent with carbon dioxide absorbed.

[15] A carbon dioxide separation and capture apparatus comprising:

an absorption apparatus comprising a mechanism for bringing a gas containing carbon dioxide into contact with the carbon dioxide absorbent according to any one of [1] to [12], to cause the carbon dioxide absorbent to absorb the carbon dioxide; and

a desorption apparatus comprising a mechanism for desorbing carbon dioxide from the carbon dioxide absorbent with carbon dioxide absorbed.

Advantageous Effects of Invention

According to the present invention, a carbon dioxide absorbent with a good carbon dioxide absorption property and particularly excellent repeated usability, a method for capturing carbon dioxide using the carbon dioxide absorbent, and a carbon dioxide separation and capture apparatus can be provided.

Brief Description of Drawing

[Fig. 1] Fig. 1 is a schematic diagram showing an embodiment of a carbon dioxide separation and capture apparatus of the present invention.

Description of Embodiments

[Definition]

In the present specification, "good carbon dioxide absorption property" means a large in the amount of carbon dioxide absorbed upon contact with a gas containing carbon dioxide, and "good absorption property for carbon dioxide from the air" means a large in the amount absorbed for carbon dioxide at a low concentration (about 0.04% by volume) from the air.

In the present specification, "good repeated usability" of a carbon dioxide absorbent means a high retention rate of an amount of carbon dioxide absorbed when cycle tests of absorption and desorption of carbon dioxide are performed.

Specifically, the amount of carbon dioxide absorbed and its retention rate can be evaluated by methods described in Examples.

"Primary amino group" means an amino group having two hydrogen atoms on a nitrogen atom, i.e., $-NH_2$ group.

In the present specification, description of "XX to YY" means "XX or more and YY or less".

[Carbon Dioxide Absorbent]

A carbon dioxide absorbent of the present invention comprises an amine compound (A) represented by the following general formula (1) and a porous material (B):

[Formula 2]

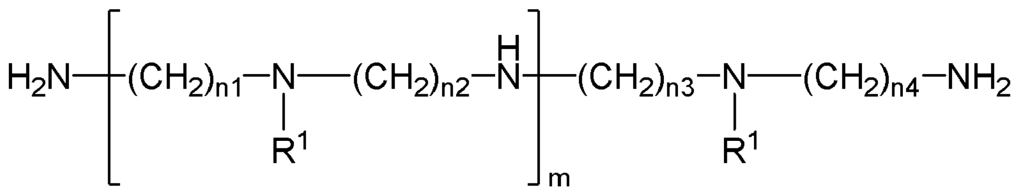

(1)

In the formula (1), each $R^1$ independently represents a hydroxy group or an organic group having 1 to 10 carbon atoms. n1 to n4 each independently represent a number of 1 to 8, and m represents a number of 0 to 10.

The carbon dioxide absorbent of the present invention, by having the above configuration, has a good carbon dioxide absorption property and particularly excellent repeated usability.

The reason why the carbon dioxide absorbent of the present invention exerts the above effects is not certain, but is considered to be as follows.

The amine compound (A) for use in the carbon dioxide absorbent of the present invention has a primary amino group with a large absorption rate for carbon dioxide and is therefore expected to exert a high carbon dioxide absorption property.

The amine compound (A) also has a tertiary amino group in the molecule and has a high proportion of the tertiary amino group to all amino groups. The tertiary amino group easily acts as a mediator for carbon dioxide without forming carbamate having strong binding force when absorbing carbon dioxide. Therefore, energy required for the desorption of carbon dioxide is considered to be small. This can further improve the repeated usability of the carbon dioxide absorbent.

The carbon dioxide absorbent contains the porous material (B) so that at least some of the amine compound (A) is supported on the porous material (B). This improves a contact area between the amine compound (A) having the carbon dioxide absorption property and a gas containing carbon dioxide, and carbon dioxide after carbon dioxide is absorbed can be desorbed (separated and captured) under a lower energy condition. Furthermore, use of the porous material (B) also suppresses the volatilization of the amine compound (A) and is expected to improve the repeated usability of the carbon dioxide absorbent.

<Amine Compound (A)>

The amine compound (A) is represented by the following general formula (1):

[Formula 3]

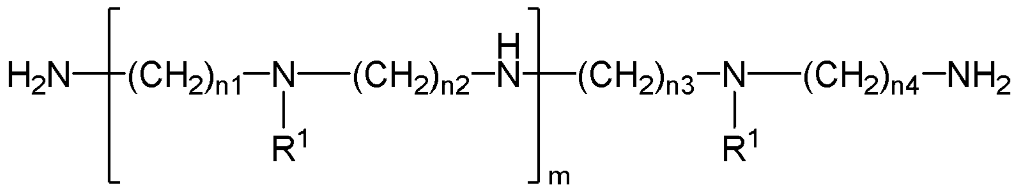

(1)

In the formula (1), each $R^1$ independently represents a hydroxy group or an organic group having 1 to 10 carbon atoms. n1 to n4 each independently represent a number of 1 to 8, and m represents a number of 0 to 10.

In the formula (1), preferably, n1 to n4 are each independently a number of 3 to 8, and m is a number of 1 to 10.

An amine compound of the formula (1) in which the numbers of methylene groups (n1 to n4 in the general formula (1)) in divalent groups adjacent to nitrogen atoms are 2 easily causes intramolecular cyclization reaction when oxidation, heating, or the like is performed. Hence, for example, if carbon dioxide is absorbed to this amine compound and subsequently heating treatment is performed for the desorption of carbon dioxide, intramolecular cyclization reaction occurs so that the amine compound is converted to a different compound, which might reduce repeated usability. By contrast, when all the numbers of methylene groups (n1 to n4 in the general formula (1)) in divalent groups adjacent to nitrogen atoms are 3 or more, the above cyclization reaction is unlikely to occur and better repeated usability is expected.

When m in the formula (1) is a number of 1 to 10, the amine compound (A) has a relatively high molecular weight and is therefore difficult to volatilize even if heating treatment is performed for the desorption of carbon dioxide after carbon dioxide is absorbed. This is also expected to contribute to improvement in repeated usability.

In the formula (1), $R^1$ each independently represents the hydroxy group or the organic group having 1 to 10 carbon atoms, preferably the hydroxy group, or a hydrocarbon group having 1 to 10 carbon atoms and optionally having a hydroxy group, an amino group, a halogen atom, a thiol group, a nitro group, a cyano group, or a carboxy group. The hydrocarbon group is preferably an alkyl group, an aryl group, or an aralkyl group, and more preferably the alkyl group.

$R^1$ is preferably the hydroxy group, or a hydrocarbon group having 1 to 6 carbon atoms and optionally having a hydroxy group, an amino group, or a cyano group, more preferably the hydroxy group, or an alkyl group having 1 to 4 carbon atoms and optionally having a hydroxy group, an amino group, or a cyano group, even more preferably an alkyl group having 1 to 2 carbon atoms and optionally having a hydroxy group, an amino group, or a cyano group, and still more preferably a methyl group, from the viewpoint of improving the carbon dioxide absorption property by improvement in amino group concentration.

A plurality of $R^1$ moieties may be the same or may be different and are preferably the same from the viewpoint of the ease of production.

In the formula (1), n1 to n4 are each independently a number of 1 to 8 and are preferably 3 to 8, more preferably 3 to 6, even more preferably 3 to 4, and still more preferably all are 3, from the viewpoint of improving the carbon dioxide absorption property by improvement in amino group concentration and from the viewpoint of improving the repeated usability. Although all of n1 to n4 may be the same or may be different, all of them are preferably the same from the viewpoint

of the ease of production.

In the formula (1), m is a number of 0 to 10. m is preferably 1 or more, more preferably 2 or more, and even more preferably 3 or more, from the viewpoint of increasing the molecular weight of the amine compound (A), suppressing volatilization, and further improving the repeated usability, and preferably 8 or less, and more preferably 6 or less, from the viewpoint of preventing a viscosity from becoming too large and fluidity from being impaired.

In the formula (1), m is an average value, and the amine compound (A) may comprise two or more compounds differing in the value of m from each other.

A total amine value of the amine compound (A) is, from the viewpoint of improving the carbon dioxide absorption property, preferably 600 mgKOH/g or more, more preferably 800 mgKOH/g or more, and even more preferably 1,000 mgKOH/g or more, and preferably 1,200 mgKOH/g or less.

A tertiary amine value of the amine compound (A) is, from the viewpoint of further improving the carbon dioxide desorption performance and the repeated usability, preferably 100 mgKOH/g or more, and more preferably 200 mgKOH/g or more, and preferably 500 mgKOH/g or less.

The total amine value represents the amount of all amino groups in the compound and refers to mg of potassium hydroxide (KOH) in an amount equal to that of an acid required for neutralizing 1 g of the compound. The tertiary amine value represents the amount of a tertiary amino group in the compound and refers to mg of potassium hydroxide (KOH) in an amount equal to that of an acid required for neutralizing the tertiary amino group in 1 g of the compound.

Specifically, the total amine value and the tertiary amine value can be determined by methods described in Examples.

In the amine compound (A), a proportion of a tertiary amino group in all amino groups is preferably 40% by mol or more from the viewpoint of improving the repeated usability, and is preferably 48% by mol or less, and more preferably 46% by mol or less, from the viewpoint of improving the carbon dioxide absorption property.

The proportion of the tertiary amino group in all amino groups is a value obtained by dividing the tertiary amine value of the amine compound (A) by the total amine value (tertiary amine value / total amine value).

In the amine compound (A), a proportion of a primary amino group in all amino groups is preferably 9.0% by mol or more, more preferably 10% by mol or more, and even more preferably 15% by mol or more, from the viewpoint of improving the carbon dioxide absorption property, and preferably 40% by mol or less, more preferably 30% by mol or less, and even more preferably 20% by mol or less, from the viewpoint of improving the repeated usability.

The proportion of the primary amino group in all amino groups is a value obtained by dividing the primary amine value of the amine compound (A) by the total amine value (primary amine value / total amine value).

Suitable specific examples of the amine compound (A) include, but are not limited to.

[Formula 4]

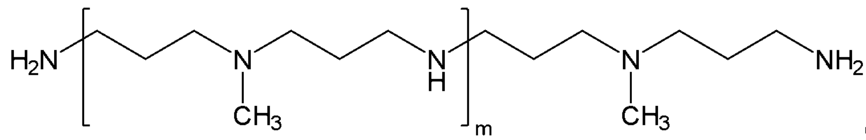

(1-1)

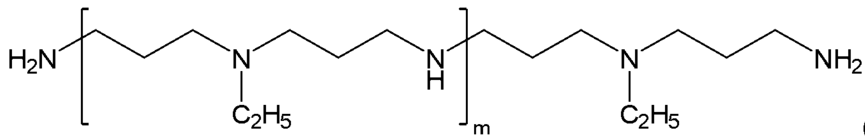

(1-2)

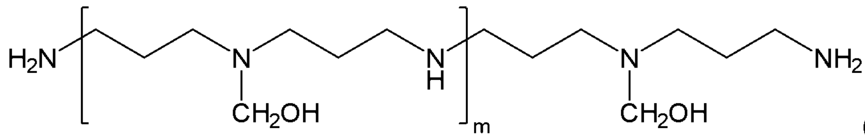

(1-3)

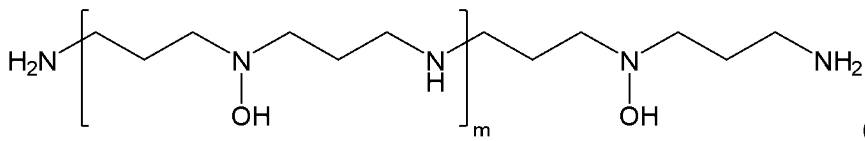

(1-4)

The amine compound (A) is preferably a compound represented by any of the above structural formulas (1-1) to (1-4), and more preferably a compound represented by the above structural formula (1-1), from the viewpoint of improving the carbon dioxide absorption property and the repeated usability and from the viewpoint of the ease of production.

A molecular weight of the amine compound (A) is, from viewpoints of suppressing weight loss by heating to

desorb carbon dioxide and further improving the repeated usability, preferably 270 or more, more preferably 400 or more, and even more preferably 500 or more, and from the viewpoint of improving the carbon dioxide absorption property, preferably 1500 or less, more preferably 1200 or less, even more preferably 1000 or less, and still more preferably 800 or less.

The molecular weight of the amine compound (A) is a weight average molecular weight (Mw) determined by tosylation method-GPC measurement described in Examples using a gel filtration chromatography (GPC) apparatus.

<Physical Properties of Amine Compound (A)>

A maximum carbon dioxide release temperature of the amine compound (A) as measured by the following method is, from viewpoints of further improving the carbon dioxide desorption performance and the repeated usability, preferably 140°C or less, more preferably 130°C or less, even more preferably 120°C or less, still more preferably 110°C or less, and yet more preferably 100°C or less. A lower limit value of the above maximum carbon dioxide release temperature is not limited, but is, e.g., 40°C or more.

(Method)

The amine compound (A) with carbon dioxide absorbed is heated from 23°C to 250°C at 10°C/minute of a heating rate, and a temperature at which an endothermic amount associated with desorption of carbon dioxide reaches a maximum is measured, and this temperature is taken as the maximum carbon dioxide release temperature. Here, the amine compound (A) with carbon dioxide absorbed can be prepared, for example, by allowing 5 mmol of the cyclic amine compound (A) to stand in the air at 23°C and 50% RH for 24 hours.

A maximum endothermic temperature of the amine compound (A) as measured by the following method is, from viewpoints of suppressing the weight loss by heating to desorb carbon dioxide and further improving the repeated usability, preferably 150°C or more, more preferably 180°C or more, even more preferably 200°C or more, and still more preferably 250°C or more.

(Method)

Heating the amine compound (A) from 23°C to 350°C at 10°C/minute of the heating rate, measuring a temperature at which an endothermic amount associated with the volatilization of the amine compound (A) reaches a maximum, and letting this temperature be the maximum endothermic temperature of the amine compound (A).

<Method for Producing Amine Compound (A)>

The amine compound (A) is obtained, for example, by subjecting a starting material amine represented by the following general formula (2) to polycondensation reaction involving deammoniation, preferably under heating and pressurization conditions.

[Formula 5]

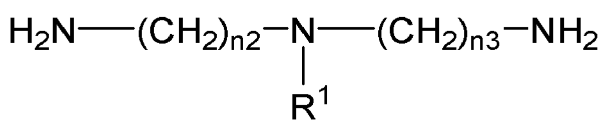

(2)

In the formula (2), $R^1$, n2, and n3 are as defined above.

In the production of the amine compound represented by the structural formula (1-1), methyliminobis(propylamine)[N,N-bis(3-aminopropyl)methylamine] can be used as the starting material amine represented by the general formula (2).

The polycondensation reaction of the starting material amine is performed, preferably in the presence of a catalyst.

As for the catalyst, a known hydrogenation catalyst can be used as a polycondensation catalyst. Examples of the catalyst that may be used include supported heterogeneous hydrogenation catalysts in which a metal such as Ni, Co, Pt, Pd, or Ru is supported on carbon, silica, alumina, diatomaceous earth, or the like; so-called Ziegler-type hydrogenation catalysts using a transition metal salt including an organic acid salt or an acetylacetone salt such as Ni, Co, Fe, or Cr, and a reducing agent such as organoaluminum; and homogeneous hydrogenation catalysts such as so-called organometallic complexes including an organometallic compound such as Ti, Ru, Rh, or Zr.

A temperature of the polycondensation reaction of the starting material amine is preferably 0 to 200°C, more preferably 10 to 180°C, and even more preferably 20 to 150°C, from the viewpoint of improving the reaction efficiency and from the viewpoint of suppressing side reaction.

A pressure of the polycondensation reaction of the starting material amine is, from the viewpoint of improving the reaction efficiency and from the viewpoint of suppressing side reaction, preferably 0.01 MPaG or more, more preferably 0.1 MPaG or more, and even more preferably 0.3 MPaG or more, and preferably 10 MPaG or less, and more preferably 3 MPaG or less. Pressurization during the polycondensation reaction is preferably performed using an inert gas such as nitrogen.

A polycondensation reaction time is not limited, but is preferably 30 minutes or longer, and more preferably 1 hour or longer, from the viewpoint of improving the molecular weight, and preferably 24 hours or shorter, more preferably 12 hours or shorter, and even more preferably 8 hours or shorter, from the viewpoint of production efficiency.

It is preferable that the above polycondensation reaction is performed in a solvent. The solvent is not limited, as long as it does not inhibit the polycondensation reaction. Examples thereof include hydrocarbon solvents including aliphatic hydrocarbons such as pentane, hexane, isopentane, heptane, octane, and isooctane; alicyclic hydrocarbons such as cyclopentane, methylcyclopentane, cyclohexane, methylcyclohexane, and ethylcyclohexane; and aromatic hydrocarbons such as toluene, ethylbenzene, and xylene. One of these solvents may be used, or two or more thereof may be used in combination.

After the reaction, the catalyst is removed from an obtained reaction liquid, and distillation and purification, or the like can be performed, if necessary, to obtain the amine compound (A).

<Porous Material (B)>

The carbon dioxide absorbent of the present invention contains the porous material (B). In the carbon dioxide absorbent, from a viewpoint that carbon dioxide can be separated and captured with lower energy, it is preferable that at least some of the amine compound (A) is supported on the porous material (B), and more preferable that the amine compound (A) is supported and solidified on the porous material (B).

The porous material (B) is preferably one that can support the amine compound (A) and withstand conditions of separating and capturing carbon dioxide, and includes, for example, porous silica, porous alumina, porous silica-alumina, porous magnesia, porous zirconia, zeolite, a zeolite analogous compound, a clay mineral, a natural mineral, activated carbon, a carbon molecular sieve (porous carbon), porous resin (synthetic adsorbent), a porous metal organic structure, and a porous waste solid, of which one or two or more can be used.

Among them, from the viewpoint of improving the carbon dioxide absorption property and the repeated usability, the porous material (B) preferably comprises at least one selected from the group consisting of porous silica and porous alumina, more preferably comprises porous silica, and even more preferably comprises mesoporous silica.

A shape of the porous material (B) is not limited, but is preferably in a particulate form, and more preferably in a spherical particulate form. The porous material (B) in a particulate form can improve a specific surface area of the porous material (B) and improve an amount of the amine compound (A) supported. This can further improve the amount of carbon dioxide absorbed.

When the porous material (B) is in a particulate form, a volume median particle size ($D_{50}$) of the porous material measured by laser diffraction/scattering particle size distribution measurement is preferably 1 μm or more, more preferably 5 μm or more, even more preferably 10 μm or more, still more preferably 20 μm or more, yet more preferably 30 μm or more, even much more preferably 40 μm or more, still much more preferably 50 μm or more, yet much more preferably 70 μm or more, even by far more preferably 80 μm or more, and still by far more preferably 90 μm or more, from the viewpoint of improving handling. It is preferably 500 μm or less, more preferably 300 μm or less, even more preferably 200 μm or less, still more preferably 180 μm or less, and yet more preferably 160 μm or less, from the viewpoint of further improving the amount of the amine compound (A) supported.

The specific surface area of the porous material (B) measured by a BET method is preferably 2 $m^2/g$ or more, more preferably 10 $m^2/g$ or more, even more preferably 30 $m^2/g$ or more, yet more preferably 50 $m^2/g$ or more, yet more preferably 70 $m^2/g$ or more, from the viewpoint of further improving the amount of the amine compound (A) supported, and preferably 3,000 $m^2/g$ or less, more preferably 1,500 $m^2/g$ or less, even more preferably 1,200 $m^2/g$ or less, and still more preferably 1,000 $m^2/g$ or less, from the viewpoint of improving the handling.

The specific surface area of the porous material (B) measured by a BET method is yet more preferably 800 $m^2/g$ or less, even much more preferably 700 $m^2/g$ or less, still much more preferably 600 $m^2/g$ or less, yet much more preferably 500 $m^2/g$ or less, and even by far more preferably 400 $m^2/g$ or less, from the viewpoint of further improving the carbon dioxide absorption property and the absorption rate for carbon dioxide of the resulting carbon dioxide absorbent.

A pore size of the porous material (B) is preferably 5 nm or more, and more preferably 10 nm or more, from the viewpoint of further improving the carbon dioxide absorption property and the absorption rate for carbon dioxide. It is preferably 200 nm or less, more preferably 100 nm or less, even more preferably 80 nm or less, and still more preferably 70

nm or less, from the viewpoint of improving the mechanical strength and the repeated usability of the porous material (B).

When the pore size of the porous material (B) for use in the carbon dioxide absorbent is 5 nm or more, the supported amine compound (A) is difficult to bury in pores of the porous material (B) and the amine compound (A) is considered to be easily adsorbed in a single-layer state to the surface of the porous material (B). This effect is expected to further improve the carbon dioxide absorption property and the absorption rate for carbon dioxide of the resulting carbon dioxide absorbent.

The pore size of the porous material (B) is even more preferably 15 nm or more, and still more preferably 20 nm or more, from the viewpoint of further improving the carbon dioxide absorption property and the absorption rate for carbon dioxide of the resulting carbon dioxide absorbent.

A pore volume of the porous material (B) is preferably 0.1 $cm^3/g$ or more, more preferably 0.3 $cm^3/g$ or more, even more preferably 0.5 $cm^3/g$ or more, and still more preferably 0.7 $cm^3/g$ or more, from the viewpoint of further improving the carbon dioxide absorption property and the absorption rate for carbon dioxide, and preferably 5.0 $cm^3/g$ or less, more preferably 3.0 $cm^3/g$ or less, and even more preferably 2.5 $cm^3/g$ or less, from the viewpoint of improving the repeated usability. Particularly, when the pore volume of the porous material (B) for use in the carbon dioxide absorbent is 0.5 $cm^3/g$ or more, the supported amine compound (A) is difficult to bury in pores of the porous material (B) and the amine compound (A) is considered to be easily adsorbed in a single-layer state to the surface of the porous material (B). This effect is expected to further improve the carbon dioxide absorption property and the absorption rate for carbon dioxide of the resulting carbon dioxide absorbent.

The pore volume of the porous material (B) is still more preferably 0.8 $cm^3/g$ or more, and yet more preferably 1.0 $cm^3/g$ or more, from the viewpoint of further improving the carbon dioxide absorption property and the absorption rate for carbon dioxide of the resulting carbon dioxide absorbent.

The specific surface area, pore size, and the pore volume of the porous material (B) can be measured, for example, by a specific surface area/pore size distribution measurement analyzer (e.g., "ASAP2020" produced by Shimadzu Corporation) using a constant volume method. As a more specific gas adsorption measurement method using the specific surface area/pore size distribution measurement analyzer, for example, a sample is pretreated by heating and vacuum evacuation, and 0.1 g of the sample for measurement is placed in a sample tube. The sample is then heated to 40°C, vacuum evacuated for 6 hours, cooled to room temperature, and mass of the sample is measured. In the measurement, a liquid nitrogen temperature is set and a pressure range is specified, and the specific surface area, the pore volume, and the pore size can be analyzed and calculated from the obtained nitrogen adsorption isotherms.

The volume median particle size ($D_{50}$), the specific surface area, the pore size, and the pore volume of the porous material (B) mean the volume median particle size ($D_{50}$), the specific surface area, the pore size, and the pore volume of the porous material (B) which is a starting material before blending into the carbon dioxide absorbent.

The porous material (B) may be granulation molded from the particulate porous material by a known method to make pellets, tablets, or other granulated products. The granulation molding method includes, for example, a dry granulation method using a compression molding machine and a wet granulation method using a binder. By using the particulate porous material granulation molded, vibration resistance and abrasion resistance can be imparted, thereby improving physical stability.

<Content>

A content of the amine compound (A) in the carbon dioxide absorbent is preferably 0.1% by mass or more, more preferably 1% by mass or more, even more preferably 10% by mass or more, still more preferably 20% by mass or more, yet more preferably 25% by mass or more, even much more preferably 30% by mass or more, still much more preferably 35% by mass or more, and yet much more preferably 40% by mass or more, from the viewpoint of improving the carbon dioxide absorption property and the repeated usability. It is preferably 90% by mass or less, more preferably 80% by mass or less, even more preferably 70% by mass or less, and still more preferably 60% by mass or less, from the viewpoint of improving the repeated usability.

The content of the amine compound (A) in the carbon dioxide absorbent is preferably 0.1 parts by mass or more, more preferably 1 part by mass or more, even more preferably 10 parts by mass or more, still more preferably 25 parts by mass or more, and yet more preferably 50 parts by mass or more, with respect to 100 parts by mass of the porous material (B) from the viewpoint of further improving the carbon dioxide absorption property and the repeated usability. It is preferably 1000 parts by mass or less, more preferably 500 parts by mass or less, even more preferably 250 parts by mass or less, still more preferably 200 parts by mass or less, and yet more preferably 150 parts by mass or less.

The content of the amine compound (A) in the carbon dioxide absorbent is even much more preferably 120 parts by mass or less, still much more preferably 100 parts by mass or less, yet much more preferably 80 parts by mass or less, and even by far more preferably 70 parts by mass or less, with respect to 100 parts by mass of the porous material (B) from the viewpoint of further improving the carbon dioxide absorption property and the absorption rate for carbon dioxide.

A total content of the amine compound (A) and the porous material (B) in the carbon dioxide absorbent is, from the

viewpoint of further improving the carbon dioxide absorption property and the repeated usability, preferably 60% by mass or more, more preferably 70% by mass or more, even more preferably 80% by mass or more, still more preferably 90% by mass or more, yet more preferably 95% by mass or more, and even much more preferably 98% by mass or more, and 100% by mass or less.

<Other Components>

The carbon dioxide absorbent of the present invention may include components other than the amine compound (A) and the porous material (B) as appropriate to the extent that the effects of the invention are not impaired. The components include, for example, an amine component other than the component (A), a deterioration inhibitor, an antifoaming agent, an antioxidant, and a desiccant to remove moisture (magnesium sulfate, molecular sieves, etc.).

A content of the amine compound (A) in all amine compounds contained in the carbon dioxide absorbent is, from the viewpoint of further improving the carbon dioxide absorption property and the repeated usability, preferably 50% by mass or more, more preferably 60% by mass or more, even more preferably 70% by mass or more, still more preferably 80% by mass or more, yet more preferably 90% by mass or more, even much more preferably 95% by mass or more, and still much more preferably 98% by mass or more, and 100% by mass or less.

A content of water in the carbon dioxide absorbent is, from the viewpoint of further improving the carbon dioxide absorption property and the repeated usability, preferably 10% by mass or less, more preferably 5% by mass or less, even more preferably 1% by mass or less, still more preferably 0.5% by mass or less, yet more preferably 0.1% by mass or less, and even much more preferably 0.01% by mass or less, and it is still much more preferable that the carbon dioxide absorbent is substantially free of water. Here, "substantially free of water" means that water is not intentionally added and does not exclude the presence of a small amount of water as an impurity.

<Burial Rate for Pore>

In the carbon dioxide absorbent of the present invention, a burial rate for pores of the carbon dioxide absorbent calculated according to the following expression is preferably 90% or less, more preferably 80% or less, even more preferably 70% or less, and still more preferably 65% or less, from the viewpoint of further improving the carbon dioxide absorption property and the absorption rate for carbon dioxide. The burial rate for pores of the carbon dioxide absorbent is preferably 10% or more, and more preferably 15% or more, from the viewpoint of improving the carbon dioxide absorption property.

Burial rate for pores (%) of the carbon dioxide absorbent = {Pore volume ($cm^3/g$) of the porous material (B) - Pore volume ($cm^3/g$) of the carbon dioxide absorbent} / Pore volume ($cm^3/g$) of the porous material (B) $\times$ 100

When the burial rate for pores of the carbon dioxide absorbent is 90% or less, a phenomenon can be circumvented in which the supported amine compound (A) blocks pores of the porous material (B) and interferes with flow paths for carbon dioxide. This is expected to further improve the carbon dioxide absorption property and the absorption rate for carbon dioxide.

The pore volume of the carbon dioxide absorbent in the above expression can be measured by the same method as in the pore volume of the porous material (B).

<Method for Preparing Carbon Dioxide Absorbent>

A method for preparing the carbon dioxide absorbent is not limited, and any known method can be used. For example, the amine compound (A) and the porous material (B) can be blended, and mixed using a known apparatus to prepare it.

When it is in an aspect in which at least some of the amine compound (A) is supported on the porous material (B), the carbon dioxide absorbent can preferably be prepared by the following method.

First, the amine compound (A), the porous material (B), and an organic solvent are blended and stirred preferably under a temperature of 5 to 60°C for 1 to 24 hours, to prepare a mixture. The organic solvent is then removed from the mixture obtained by distillation and the like, and the remaining solids are dried under reduced pressure to obtain the carbon dioxide absorbent.

As the organic solvent, from a viewpoint of dispersibility of the amine compound (A) and the porous material (B) and from a viewpoint of easy removal from the carbon dioxide absorbent, preferable is a monovalent alcohol having 4 or less of carbon atoms, and more preferable is at least one selected from the group consisting of methanol, ethanol, and isopropyl alcohol.

The carbon dioxide absorbent of the present invention can be suitably used in technology of directly absorbing carbon dioxide (DAC) from the air because of its good absorption performance for carbon dioxide from the air.

The carbon dioxide absorbent of the present invention can be also suitably used in case of capturing carbon dioxide at low concentrations of, e.g., 0.01% by volume or more and 1% by volume or less.

The carbon dioxide absorbent of the present invention particularly has good repeated usability and has a high retention rate of the amount of carbon dioxide absorbed even if the cycle of absorption and desorption of carbon dioxide is repeated. For the carbon dioxide absorbent, a larger initial amount of carbon dioxide absorbed is more preferable, whereas a low retention rate of the amount of carbon dioxide absorbed in repeated use is industrially disadvantageous because the numbers of times of exchange and disposal of the carbon dioxide absorbent are increased. Thus, the carbon dioxide absorbent with good repeated usability is regarded as having high industrial applicability.

[Method for Capturing Carbon Dioxide]

A method for capturing carbon dioxide (hereinafter simply referred to as "the method of the present invention", too) of the present invention is characterized by comprising using the above carbon dioxide absorbent. According to the method of the present invention, the amount of carbon dioxide absorbed from a gas containing carbon dioxide can be improved. It also enables to separate and capture carbon dioxide at lower energy, and the repeated usability of the carbon dioxide absorbent is also good.

It is preferable that the method for capturing carbon dioxide of the present invention comprises a step of bringing the gas containing carbon dioxide into contact with the carbon dioxide absorbent, to cause the carbon dioxide absorbent to absorb carbon dioxide (absorption step).

<Absorption Step>

The absorption step is a step of bringing the gas containing carbon dioxide into contact with the carbon dioxide absorbent, to cause the carbon dioxide absorbent to absorb the carbon dioxide. A contact method of the carbon dioxide absorbent and the gas can be selected as suitable one depending on morphology of the carbon dioxide absorbent and is not limited. For example, by passing the gas containing carbon dioxide through the carbon dioxide absorbent, spraying the carbon dioxide absorbent in the gas containing carbon dioxide, or locating the carbon dioxide absorbent in the gas containing carbon dioxide, the gas containing carbon dioxide can be brought into contact with the carbon dioxide absorbent.

The gas containing carbon dioxide is not limited, but includes, for example, air, thermal power plant exhaust gas, steel plant exhaust gas, cement plant exhaust gas, chemical plant exhaust gas, bio-fermentation gas, and natural gas. There is a need for capturing carbon dioxide from these gases with particularly energy savings, and the present invention is particularly effective. A concentration of carbon dioxide in the gas, pressure of the gas, and a temperature of the gas are not limited, and the method of the present invention can be applied in gases with a wide range of conditions.

In addition, the gas containing carbon dioxide may also contain acidic gas and the like other than carbon dioxide. The acidic gas includes CO, NOx, and SOx in the exhaust gas, formaldehyde come up from methanol-fueled power generation, and other hydrogen chloride, hydrogen sulfide. When the above gas containing carbon dioxide contains acidic gases and the like other than carbon dioxide, it is preferable that known steps for removing other acidic gases are combined. Specifically, included is an aspect that the method for capturing carbon dioxide of the present invention is applied to the gas containing acidic gases and the like other than carbon dioxide, or an aspect that the method for capturing carbon dioxide of the present invention is applied after removing other acidic gases from the gas containing acidic gases and the like other than carbon dioxide by a known means.

In the absorption step, a temperature when the gas containing carbon dioxide is brought into contact with the carbon dioxide absorbent is preferably 0°C or more and less than 60°C, more preferably 20°C or more and less than 60°C, and even more preferably 30°C or more and less than 60°C, from the viewpoint of improving the amount of carbon dioxide absorbed.

More preferably, the method of the present invention comprises the absorption step of bringing the gas containing carbon dioxide into contact with the carbon dioxide absorbent, to cause the carbon dioxide absorbent to absorb the carbon dioxide, and a desorption step of desorbing carbon dioxide from the carbon dioxide absorbent with carbon dioxide absorbed in the absorption step, wherein the desorption step comprises at least one step selected from the group consisting of the following (I) to (III). By this method, carbon dioxide can be separated and captured from the gas containing carbon dioxide.

(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and

(III) a step of heating the carbon dioxide absorbent with carbon dioxide absorbed.

<Desorption Step>

A desorption step is a step of desorbing carbon dioxide from the carbon dioxide absorbent with carbon dioxide absorbed in the absorption step. The method for desorbing carbon dioxide from the carbon dioxide absorbent with carbon dioxide absorbed preferably includes a method comprising at least one step selected from the group consisting of the above (I) to (III). Two or more of the steps (I) to (III) may be combined.

In the (I) step of subjecting the carbon dioxide absorbent with carbon dioxide absorbed to the reduced pressure condition (hereinafter referred to as "Step (I)", too), the reduced pressure condition is preferably 10 kPa or less, more preferably 5 kPa or less, even more preferably 1 kPa or less, from a viewpoint of improving carbon dioxide separating and capturing efficiency. In addition, from a viewpoint of inhibiting volatilization of the amine compound (A) in the carbon dioxide absorbent, it is preferably 0.1 kPa or more.

In Step (I), a temperature when the carbon dioxide absorbent is subjected to the reduced pressure condition is not limited, but from the viewpoint of inhibiting the volatilization of the amine compound (A) in the carbon dioxide absorbent, it is preferably less than 50°C, more preferably 45°C or less. In addition, from the viewpoint of improving the carbon dioxide separating and capturing efficiency, it is preferably 0°C or more, and more preferably 10°C or more.

In the (II) step of bringing the inert gas not containing carbon dioxide into contact with the carbon dioxide absorbent with carbon dioxide absorbed (hereinafter referred to as "Step (II)", too), reducing a partial pressure of carbon dioxide allows to promote the desorption of carbon dioxide. The inert gas not containing carbon dioxide includes nitrogen, helium, argon, and one or more of these gases can be used. Above all, from the viewpoint of improving the carbon dioxide separating and capturing efficiency, the inert gas not containing carbon dioxide is preferably at least one selected from the group consisting of the nitrogen and the argon.

In Step (II), the method for bringing the inert gas not containing carbon dioxide into contact with the carbon dioxide absorbent includes the same method as the contact method described in the absorption step.

In Step (II), a temperature when the inert gas not containing carbon dioxide is brought into contact with the carbon dioxide absorbent is not limited, and heating as specified in the step (III) may be simultaneously performed in Step (II), or may be at a temperature of room temperature or less. From the viewpoint of inhibiting the volatilization of the amine compound (A) in the carbon dioxide absorbent, the temperature is preferably less than 50°C, and more preferably 45°C or less. In addition, from the viewpoint of improving the carbon dioxide separating and capturing efficiency, the temperature is preferably 0°C or more, and more preferably 10°C or more.

A heating temperature in the (III) step of heating the carbon dioxide absorbent with carbon dioxide absorbed (hereinafter referred to as "Step (III)", too) is preferably 50°C or more and 120°C or less, more preferably 55°C or more and 110°C or less, and even more preferably 60°C or more and 100°C or less, from the viewpoint of improving the carbon dioxide separating and capturing efficiency.

The heating in Step (III) can be performed by a known method using apparatus equipped with a heating means. A heating method includes, for example, heating with steam or a heating medium, hot air heating, electromagnetic wave heating, ultrasonic heating, and induction heating.

The carbon dioxide absorbent and the carbon dioxide which are separated in the desorption step can be captured separately and reused.

[Carbon Dioxide Separation and Capture Apparatus]

The carbon dioxide separation and capture apparatus of the present invention (hereinafter referred to as "the apparatus of the present invention", too) comprises an absorption apparatus comprising a mechanism for bringing the gas containing carbon dioxide into contact with the carbon dioxide absorbent, to cause the carbon dioxide absorbent to absorb the carbon dioxide, and a desorption apparatus comprising a mechanism for desorbing carbon dioxide from the carbon dioxide absorbent with carbon dioxide absorbed.

The apparatus of the present invention will be described with reference to Fig. 1. Fig. 1 is a schematic diagram showing an embodiment of a carbon dioxide separation and capture apparatus 100 of the present invention, in which 1 denotes the absorption apparatus and 2 denotes the desorption apparatus in Fig. 1.

<Absorption Apparatus>

The absorption apparatus 1 in the carbon dioxide separation and capture apparatus 100 is equipped with the carbon dioxide absorbent and comprises a mechanism for bringing the gas containing carbon dioxide into contact with the carbon dioxide absorbent, to cause the carbon dioxide absorbent to absorb the carbon dioxide.

The absorption apparatus 1 is not limited, as long as it has configuration such that the gas containing carbon

dioxide are brought into contact with the carbon dioxide absorbent, depending on the morphology of the carbon dioxide absorbent. As shown in Fig. 1, for example, the absorption apparatus 1 can be equipped with an absorbent holding part 12 that holds a carbon dioxide absorbent 12a inside a reaction column 11 and can be further equipped with a gas supplying part 13 that supplies the gas containing carbon dioxide to the absorbent holding part 12. In addition, the absorption apparatus 1 can be also equipped with an absorbent discharging part (not shown) that discharges the carbon dioxide absorbent 12a held in the absorbent holding part 12 and an absorbent supplying part (not shown) that supplies a new carbon dioxide absorbent to the absorbent holding part 12, from a viewpoint of discharging the carbon dioxide absorbent with carbon dioxide absorbed from the absorbent holding part 12 and supplying a new carbon dioxide absorbent.

The absorption apparatus 1 may further comprise a heating and cooling mechanism to adjust the temperature when the gas containing carbon dioxide is brought into contact with the carbon dioxide absorbent. It may also comprise a carbon dioxide concentration measuring mechanism so that a carbon dioxide concentration in the gas can be measured. Furthermore, the absorption apparatus 1 may comprise a pressurizing and depressurizing mechanism to adjust pressure when the gas containing carbon dioxide is brought into contact with the carbon dioxide absorbent.

The carbon dioxide separation and capture apparatus 100 may have a connecting part 3 for supplying the carbon dioxide absorbent with carbon dioxide absorbed in the absorption apparatus 1 to the desorption apparatus 2.

A means of supplying the carbon dioxide absorbent with carbon dioxide absorbed from the absorption apparatus 1 to the desorption apparatus 2 is not limited, and the absorption apparatus 1 operated for a certain period of time may be stopped once, and the carbon dioxide absorbent in the reaction column 11 equipped in the absorption apparatus 1 may be supplied to the desorption apparatus 2 together. Alternatively, from the absorbent holding part 12 of the absorption apparatus 1, the carbon dioxide absorbent may be supplied continuously or interruptedly to the desorption apparatus 2 by utilizing the connecting part 3.

<Desorption Apparatus>

The desorption apparatus 2 in the carbon dioxide separation and capture apparatus 100 comprises a mechanism for desorbing carbon dioxide from the carbon dioxide absorbent with carbon dioxide absorbed in the absorption apparatus 1. The desorption apparatus 2 is not limited as long as it comprises a mechanism for desorbing carbon dioxide from the carbon dioxide absorbent with carbon dioxide absorbed in the absorption apparatus 1, but it is preferable that it comprises a mechanism to perform at least one step of the above (I) to (III). Examples of the mechanism include a depressurizing mechanism, an inert gas supplying mechanism, and a heating mechanism.

As shown in Fig. 1, for example, the desorption apparatus 2 can be equipped with an absorbent holding part 22 that holds a carbon dioxide absorbent 22a with carbon dioxide absorbed inside a reaction column 21, and further equipped with a gas discharging part 23 that discharges carbon dioxide desorbed from the carbon dioxide absorbent. The desorption apparatus 2 also comprises at least one mechanism (not shown) selected from a depressurizing mechanism for bringing the inside of the reaction column 21 under a reduced pressure condition, an inert gas supplying mechanism for supplying the inert gas to the absorbent holding part 22, and a heating mechanism for heating the absorbent holding part 22.

In addition to the depressurizing mechanism, the inert gas supplying mechanism, and the heating mechanism described above, the desorption apparatus 2 may comprise the carbon dioxide concentration measuring mechanism or the like, in the same manner as the absorption apparatus 1.

The carbon dioxide absorbent after carbon dioxide is desorbed in the desorption apparatus 2 can also be reused by supplying it back into the absorption apparatus 1 again from an absorbent discharging part 24 for supplying the carbon dioxide absorbent after carbon dioxide is desorbed to the absorption apparatus 1.

The carbon dioxide separation and capture apparatus 100 may further comprise a capturing apparatus for capturing carbon dioxide desorbed. Still, here, the carbon dioxide captured may be used for agricultural applications such as a petroleum promoted capturing method and a plant factory; industrial gas applications such as beverages and welding; chemical synthesis applications; and carbon dioxide capture and storage (CCS) applications. Prior to use in these applications, the carbon dioxide captured may also be concentrated.

## Examples

Hereinafter, the present invention will be described with reference to examples, but the present invention is not limited to the scope of the examples. In the present examples, various measurements and evaluations were performed by the following methods.

(Total Amine Value of Amine Compound)

The total amine value was measured by the following method in accordance with JIS K7237-1995.

(1) Dissolving 0.1 g of the amine compound in 20 mL of the acetic acid.
(2) Determining the total amine value by titrating the solution obtained in the above (1) with the 0.1 N perchloric acid-acetic acid solution using the automatic potentiometric titrator ("AT-610" produced by Kyoto Electronics Manufacturing Co., Ltd.).

(Tertiary Amine Value of Amine Compound)

The tertiary amine value was measured by the following method in accordance with ASTM D-2073.

(1) Precisely weighing 0.5 g of the amine compound into a 110 mL screw tube, adding 25 mL of acetic anhydride and 2 mL acetic acid thereto, and covering the opening of the screw tube with a parafilm.
(2) Heating the screw tube of (1) at 130°C for 45 minutes on a hot plate.
(3) Determining the tertiary amine value by titrating the solution obtained in the above (2) with the 0.1 N perchloric acid-acetic acid solution using the automatic potentiometric titrator.

(Molecular Weight of Amine Compound)

The molecular weight of the amine compound was determined by the following tosylation method-GPC measurement.
0.02 g of the amine compound was weighed into a vial for a block thermostat and adjusted to 1 mL by the addition of trifluoroacetic anhydride. The vial was tightly sealed and heated for 1.1 hours in a block thermostat set to 80°C. After being allowed to cool to room temperature, the vial was opened and heated again for 1.1 hours in the block thermostat of 80°C to volatilize an excess of trifluoroacetic anhydride. After the vial was allowed to cool to room temperature, 1 mL of a solution of chloroform/methanol = 85/15 (v/v) was added thereto, and the vial was allowed to stand for about 1 hour for dissolution. After complete dissolution was visually confirmed, the solution was filtered through a non-aqueous filter with a pore size of 0.45 μm, and GPC measurement was performed with a filtrate as a measurement sample under the following conditions to determine a weight average molecular weight of the amine compound.

[Measurement Conditions]

GPC apparatus: "Prominence" produced by Shimadzu Corporation
Column: connected columns of "KF-403HQ" and "KF-404HQ" produced by Shodex/Resonac Corporation
Measurement temperature: 40°C
Eluent: THF
Detector: RI
Standard sample: Polystyrene PS (Mp: 162 to 136000, produced by Agilent Technologies, Inc.)

(Maximum Endothermic Temperature of Amine Compound)

DSC measurements were performed on the amine compounds to measure the maximum endothermic temperature of the amine compounds as follows. First, a differential scanning calorimetry was performed on the amine compounds using a differential thermogravimetric analyzer ("DTG-60" produced by Shimadzu Corporation) under conditions of 23 to 350°C of a measurement temperature range, 10°C/minute of a heating rate, and a nitrogen atmosphere. From the DSC curve obtained thereby, the temperature at which the endothermic amount associated with the volatilization of the amine compound reached the maximum was identified, and the temperature thereof was used as the maximum endothermic temperature of the amine compound.

(Maximum Carbon Dioxide ($CO_2$) Release Temperature of Amine Compound)

A carbon dioxide concentration meter and a petri dish were placed inside an openable desiccator (Internal Dimension: 370 mm x 260 mm x 272 mm). The amine compound (5 mmol) was added to the petri dish in the desiccator, a door was immediately closed, and the amine compound was stood in the desiccator for 24 hours under an air environment of 23°C and 50% RH. An initial concentration of carbon dioxide was adjusted to about 400 ppm.
Then, the amine compound was removed from the desiccator to obtain the amine compound with carbon dioxide absorbed. The DSC measurement was performed on the amine compound with carbon dioxide absorbed as follows, to measure the maximum carbon dioxide release temperature of the amine compound. First, the differential scanning

calorimetry was performed on the amine compound using the differential thermogravimetric analyzer ("DTG-60" produced by Shimadzu Corporation) under the conditions of 23 to 250°C of a measurement temperature range, 10°C/minute of the heating rate, and the nitrogen atmosphere. From the DSC curve obtained thereby, the temperature at which the endothermic amount associated with the desorption of carbon dioxide reached the maximum was identified, and the temperature thereof was used as the maximum carbon dioxide release temperature of the amine compound.

(Volume Median Particle Size ($D_{50}$) of Porous Material)

Using a laser diffraction/scattering particle size distribution analyzer ("LMS-200e" produced by Malvern Panalytical Ltd.,), particle distribution of the porous material was measured.

Then, the particle diameter corresponding to 50% of a cumulative volume frequency calculated from the smaller particle diameter of the particle distribution was taken as the volume median particle size ($D_{50}$) of the porous material.

(Specific Surface Areas, Pore Sizes, and Pore Volumes of Porous Material and Carbon Dioxide Absorbent)

The specific surface areas, the pore sizes, and the pore volumes of the porous material and the carbon dioxide absorbent were measured by the specific surface area/pore size distribution measurement analyzer ("ASAP2020" produced by Shimadzu Corporation).

(Burial Rate for Pore of Carbon Dioxide Absorbent)

The burial rate for pores of the carbon dioxide absorbent was calculated according to the following expression using the values of the pore volumes of the porous material and the carbon dioxide absorbent measured by the method.

Burial rate for pores (%) of the carbon dioxide absorbent = {Pore volume ($cm^3/g$) of the porous material (B) - Pore volume ($cm^3/g$) of the carbon dioxide absorbent} / Pore volume ($cm^3/g$) of the porous material (B) $\times$ 100

(Evaluation 1 of carbon Dioxide Absorption Capacity and Repeated Usability of Carbon Dioxide Absorbent)

The carbon dioxide absorption capacity and the repeated usability of the carbon dioxide absorbent described in Table 1 were evaluated by the following method.

To a glass container with nitrogen replacement, 300 mg of the amine compound, 10 g of methanol, and 300 mg of the porous material were added and stirred for 10 hours to homogenize them. The resulting mixture was then placed under a circumstance of 40°C and 100 hPa, to distil the methanol off, followed by vacuum drying at room temperature (23°C) for 24 hours to obtain the carbon dioxide absorbent.

Then, 15 mg of the carbon dioxide absorbent obtained was set in a differential thermogravimetric analyzer ("EXSTER TGD6200" produced by Hitachi High-Tech Corporation) and stood at 45°C under a dry air environment for 6 hours, and an increased amount in mass of the solid carbon dioxide absorbent was measured. Here, as a gas used at measurements, air (flow rate: 200 mL/min) and nitrogen (flow rate: 200 mL/min) were used at absorbing carbon dioxide and desorbing carbon dioxide, respectively. From the increased amount in the mass of the carbon dioxide absorbent, the amount of carbon dioxide absorbed of the carbon dioxide absorbent was calculated (1st time). A unit of the amount of carbon dioxide absorbed in Table 1 is an amount of carbon dioxide (mg) absorbed per 1 g of the carbon dioxide absorbent.

After the 1st time evaluation of the carbon dioxide absorption capacity was completed, the carbon dioxide absorbent was removed from the analyzer and the carbon dioxide absorbent with carbon dioxide absorbed was heated at 120°C for 30 minutes to desorb the carbon dioxide absorbed, regenerating the carbon dioxide absorbent.

Then, on the carbon dioxide absorbent regenerated, the above evaluation of the carbon dioxide absorption capacity was performed again, to measure an amount of carbon dioxide absorbed (2nd time).

Then, the carbon dioxide absorbent was removed from the analyzer and the carbon dioxide absorbent with carbon dioxide absorbed was heated at 120°C for 30 minutes to desorb the carbon dioxide absorbed, regenerating the carbon dioxide absorbent again.

Then, on the carbon dioxide absorbent regenerated, the above evaluation of the carbon dioxide absorption capacity was performed again, to measure the amount of carbon dioxide absorbed (3rd time).

Here, the retention rate of the amount of carbon dioxide absorbed was also calculated based on the amount of carbon dioxide absorbed of the 1st time.

(Evaluation of Carbon Dioxide Absorption Capacity and Repeated Usability of Carbon Dioxide Absorbent - 2: Evaluation under Practical Application Conditions)

The carbon dioxide absorption capacity and repeated usability under practical application conditions of the carbon dioxide absorbent described in Tables 2 and 3 were evaluated by the following method.

The carbon dioxide absorbents capture carbon dioxide from a gas mixture containing moisture in addition to carbon dioxide such as under an air environment that includes outdoor air and indoor space or combustion exhaust gas. Therefore, the amount of carbon dioxide absorbed and the amount of carbon dioxide desorbed under humidity-controlled conditions, taking into account the effect of moisture in the gas, were measured using a catalyst analyzer ("BELCATII" produced by MicrotracBELL Corporation) by the following method, to evaluate the carbon dioxide absorption capacity and repeated usability of the carbon dioxide absorbent.

(Evaluation 2-1: Heating Cycle Evaluation of Carbon Dioxide Absorbent)

(1) To the glass container with nitrogen replacement, 300 mg of the amine compound, 10 g of methanol, and 300 mg of the porous material were added and stirred for 10 hours to homogenize them. The resulting mixture was then placed under the circumstance of 40°C and 100 hPa, to distil the methanol off, followed by the vacuum drying at room temperature (23°C) for 24 hours to obtain the carbon dioxide absorbent.
(2) Then, 200 mg of the carbon dioxide absorbent obtained was charged into a reaction tube of the above catalyst analyzer, and heated at 100°C for 1 hour in a nitrogen stream (flow rate: 100 mL/min) to perform dry and degas pretreatment, and then the reaction tube was kept at 40°C. Then, the introducing gas was switched to a 400 ppm of carbon dioxide/nitrogen gas mixture (total flow rate: 1,000 mL/min) humidity-controlled to 40°C and 40% RH (absorption step), and at the same time, a change in an outlet gas composition of the catalyst analyzer over time was measured by a gas mass spectrometer (BELMASS; produced by MicrotracBELL Corporation), to obtain a breakthrough curve. After the amount of carbon dioxide absorbed reached saturation, the reaction tube was heated to 80°C along with switching the introducing gas to nitrogen (flow rate: 500 mL/min) (desorption step), and the change in the outlet gas composition of the catalyst analyzer over time was subsequently measured by the gas mass spectrometer.
The amount of carbon dioxide absorbed in the carbon dioxide absorbent was calculated from, the time from a start of absorption until saturation was reached, and an accumulated value of an outlet concentration change of carbon dioxide, and is shown in Table 2 (1st time evaluation of the carbon dioxide absorption capacity). The amount of carbon dioxide desorbed from the carbon dioxide absorbent was calculated from, the time from when the introducing gas was switched to nitrogen until carbon dioxide was almost undetectable from the outlet side, and the accumulated value of the outlet concentration change of carbon dioxide.
(3) After the 1st time evaluation of the carbon dioxide absorption capacity was completed, with respect to the carbon dioxide absorbent regenerated by performing the above desorption step, an operation of the above (2) was further repeated 9 times, resulting in a total of 10 times of carbon dioxide absorption and desorption steps. Based on the amount of carbon dioxide absorbed in the 1st time evaluation, the retention rate of the amount of carbon dioxide absorbed in the 5th and 10th evaluations were calculated.

(Evaluation 2-2: Reduced Pressure Cycle Evaluation of Carbon Dioxide Absorbent)

(1) After 100 mg of the carbon dioxide absorbent prepared by the same method as described above was weighed and charged in the reaction tube of the above catalyst analyzer, a temperature of the absorbent was held at 60°C to perform pretreatment by pressure reducing exhaust for 1 hour. Then, the reaction tube was kept at 40°C, and the introducing gas was switched to the 400 ppm of carbon dioxide/nitrogen gas mixture (total flow rate: 1,000 mL/min) humidity-controlled to 40°C and 40% RH (absorption step), and at the same time, a change in the outlet gas composition of the catalyst analyzer over time was measured by the gas mass spectrometer ("BELMASS" produced by MicrotracBELL Corporation), to obtain a breakthrough curve. The amount of carbon dioxide absorbed in the carbon dioxide absorbent was calculated from, the time from the start of absorption until the saturation was reached, and the accumulated value of the outlet concentration change of carbon dioxide, and is shown in Table 3 (1st time evaluation of the carbon dioxide absorption capacity).
(2) The amount of carbon dioxide desorbed due to the reduced pressure in the carbon dioxide absorbent was defined as the amount of carbon dioxide absorbed in a case of using the carbon dioxide absorbent after desorbing carbon

dioxide, since the amount of carbon dioxide equivalent to the carbon dioxide desorbed due to the reduced pressure would be absorbed afterward. Specifically, the absorbent with carbon dioxide absorbed in the above (1) was kept at 40°C and subjected to the pressure reduced exhaust as it is for 30 minutes by a manual operation using a vacuum pump (desorption step), and then, again, by the same method as the above (1), the 400 ppm of carbon dioxide/nitrogen gas mixture was introduced, and the changes in the outlet gas composition over time were obtained by measuring them by the gas mass spectrometer ("BELMASS" produced by MicrotracBELL Corporation). Still, the attained vacuum at a time of pressure reduction for 30 minutes was 0.5 kPa.

(3) After the 1st time evaluation of the carbon dioxide absorption capacity was completed, with respect to the carbon dioxide absorbent regenerated by performing the desorption step described in the above (2), the step of the above (1) and the desorption step were further repeated 9 more times, resulting in the total of 10 times of the carbon dioxide absorption and desorption steps. Based on the amount of carbon dioxide absorbed in the 1st time evaluation, the retention rates of the amount of carbon dioxide absorbed in the 5th time and 10th time evaluations were calculated.

Still, a unit of the amount of carbon dioxide absorbed in Tables 2 and 3 is the amount of carbon dioxide absorbed (mg) per 1 g of the carbon dioxide absorbent.

Synthesis Example 1: Production of directly condensed oligomer of methyliminobis(propylamine) (P-MIBPA)

An autoclave (capacity of 150 mL, material: SUS316L) equipped with a stirrer and a heater was charged with 0.80 g of a Raney nickel catalyst (produced by W. R. Grace & Co.), 35 g of a solvent toluene (produced by Kanto Chemical Co., Inc.), and 8.0 g of methyliminobis(propylamine). The autoclave was purged with nitrogen (1.0 MPa $\times$ 4), pressurized with nitrogen so as to attain an internal pressure of 1.5 MPa, and stirred at a temperature of 140°C for 4 hours. Subsequently, the autoclave was cooled to room temperature, and a reaction liquid was filtered under increased pressure to obtain a mixed solution of the starting material methyliminobis(propylamine) and a condensed oligomer. After the solvent was distilled off by an evaporator, unreacted methyliminobis(propylamine) was further removed at a boiling point of 130°C/20 mmHg by distillation under reduced pressure to obtain 4.6 g of P-MIBPA (Mw: 722) represented by the following structural formula:

[Formula 6]

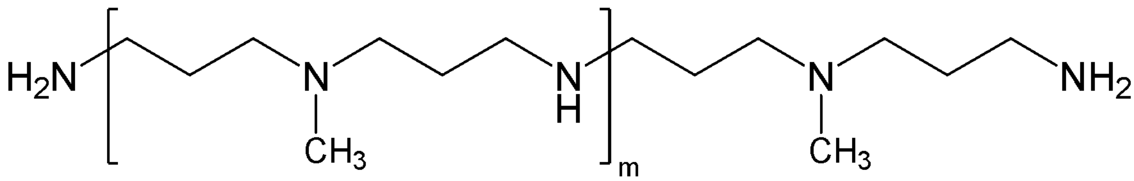

In the above formula, m calculated from the Mw of the P-MIBPA is 4.5.

The P-MIBPA has a total amine value of 1029 and a tertiary amine value of 413, i.e., the P-MIBPA has about 40% by mol of a tertiary amino group with respect to all amino groups.

Example 1 and Comparative Examples 1 and 2: Preparation and Evaluation 1 of Carbon Dioxide Absorbent

Using the amine compound and the porous material shown in Table 1, a carbon dioxide absorbent was prepared and evaluated by the method described in the section "Evaluation 1 of Carbon Dioxide Absorption Capacity and Repeated Usability of Carbon Dioxide Absorbent". The results are shown in Table 1.

Table 1

| | Amine Compound | | | | | | | Porous Material (B) | Evaluation 1 of $CO_2$ Absorption Capacity and Repeated Usability | | | | | |
|---|---|---|---|---|---|---|---|---|---|---|---|---|---|---|
| | Name | Structural Formula | Molecular Weight [-] | Maximum $CO_2$ Release Temperature [°C] | Maximum Endothermic Temperature [°C] | Total amine value [mgKOH/g] | Tertiary amine value [mgKOH/g] | Name | Amount of $CO_2$ Absorbed [mg-$CO_2$/g] | | | Retention Rate of Amount of $CO_2$ Absorbed [%] | | |
| | | | | | | | | | 1st Time | 2nd Time | 3rd Time | 1st Time | 2nd Time | 3rd Time |
| Example 1 | P-MIBPA | 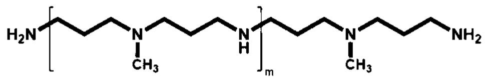 | 722 | 96.3 | 288.3 | 1029 | 413 | Mesoporous Silica SBA15 | 45.9 | 45.8 | 45.5 | 100.0 | 99.8 | 99.1 |
| Comparative Example 1 | TEPA | 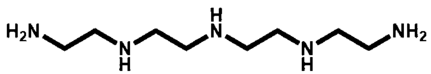 | 189.3 | 103.7 | 188.6 | 1486 | 0 | Mesoporous Silica SBA15 | 47.6 | 47.0 | 42.6 | 100.0 | 98.6 | 89.5 |
| Comparative Example 2 | PEI | 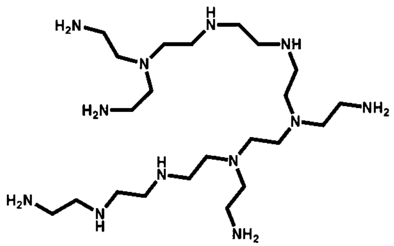 | 600 | 88.5 | 254.2 | 1554 | 323 | Mesoporous Silica SBA15 | 49.7 | 48.3 | 43.4 | 100.0 | 97.2 | 91.1 |

Example 2 and Comparative Examples 3 and 4: Preparation and Evaluation under Practical Application Condition of Carbon Dioxide Absorbent

Using the amine compound and the porous material shown in Table 2, a carbon dioxide absorbent was prepared and evaluated by the method described in "Evaluation 2-1" of the section "Evaluation 2 of Carbon Dioxide Absorption Capacity and Repeated Usability of Carbon Dioxide Absorbent". The results are shown in Table 2.

Example 3 and Comparative Examples 5 and 6: Preparation and Evaluation under Practical Application Condition of Carbon Dioxide Absorbent

Using the amine compound and the porous material shown in Table 3, a carbon dioxide absorbent was prepared and evaluated by the method described in "Evaluation 2-2" of the section "Evaluation 2 of Carbon Dioxide Absorption Capacity and Repeated Usability of Carbon Dioxide Absorbent". The results are shown in Table 3.

[Table 2]

Table 2

| | Amine compound | Porous Material (B) | Absorption Step | Desorption Step | Evaluation 2-1 of $CO_2$ Absorption Capacity and Repeated Usability | | | | | |
|---|---|---|---|---|---|---|---|---|---|---|
| | | | | | Amount of $CO_2$ Absorbed [mg-$CO_2$/g] | | | Retention Rate of Amount of $CO_2$ Absorbed [%] | | |
| | Name | Name | $CO_2$ Absorption Condition | $CO_2$ Desorption Condition | 1st Time | 5th Time | 10th Time | 1st Time | 5th Time | 10th Time |
| Example 2 | P-MIBPA | Mesoporous Silica SBA15 | 400 ppm $CO_2/N_2$ Gas Mixture, 40°C, 40%RH, 2 hours | 80°C/Normal Pressure, 30 minutes | 78.9 | 78.2 | 77.6 | 100 | 99.1 | 98.4 |
| Comparative Example 3 | TEPA | Mesoporous Silica SBA15 | | | 76.3 | 64.3 | 55.7 | 100 | 84.3 | 73.0 |
| Comparative Example 4 | PEI | Mesoporous Silica SBA15 | | | 81.2 | 71.5 | 66.2 | 100 | 88.1 | 81.5 |

[Table 3]

Table 3

| | Amine compound | Porous Material (B) | Absorption Step | Desorption Step | Evaluation 2-2 of $CO_2$ Absorption Capacity and Repeated Usability | | | | | |
|---|---|---|---|---|---|---|---|---|---|---|
| | | | | | Amount of $CO_2$ Absorbed [mg-$CO_2$/g] | | | Retention Rate of Amount of $CO_2$ Absorbed [%] | | |
| | Name | Name | $CO_2$ Absorption Condition | $CO_2$ Desorption Condition | 1st Time | 5th Time | 10th Time | 1st Time | 5th Time | 10th Time |
| Example 3 | P-MIBPA | Mesoporous Silica SBA15 | 400 ppm $CO_2/N_2$ Gas Mixture, 40°C, 40%RH, 2 hours | 40°C/0.5 kPa, 30 minutes | 58.7 | 57.3 | 56.3 | 100 | 97.6 | 95.9 |
| Comparative Example 5 | TEPA | Mesoporous Silica SBA15 | | | 76.3 | 64.3 | 55.7 | 100 | 84.3 | 73.0 |
| Comparative Example 6 | PEI | Mesoporous Silica SBA15 | | | 81.2 | 74.3 | 65.7 | 100 | 91.5 | 80.9 |

In Examples and Comparative Examples described in Tables 1 to 3, as the amine compounds and the porous materials, the following were used.

(Amine Compound)
P-MIBPA: the amine compound obtained in Synthesis Example 1
TEPA: tetraethylenepentamine (produced by Tokyo Chemical Industry Co., Ltd.)
PEI: polyethyleneimine having the structure described in Table 1 (average molecular weight: 600; produced by FUJIFILM Wako Pure Chemical Corporation)
(Porous Material)
Mesoporous Silica SBA15 (produced by Merck KGaA)

The volume median particle size ($D_{50}$): 100 μm, the specific surface area by the BET method: 800 $m^2$/g, the pore size: 10 nm, the pore volume: 0.8 $cm^3$/g

From Table 1, it is evident that the carbon dioxide absorbent of the present invention has a high retention rate of the carbon dioxide absorption capacity and therefore has excellent repeated usability. From Tables 2 and 3, it is evident that the carbon dioxide absorbent of the present invention has a high retention rate of the carbon dioxide absorption capacity and good repeated usability if used repeatedly using a gas mixture containing steam in addition to carbon dioxide.

Next, in Tables 5 to 7, evaluation was made on the correlation of the pore size and the pore volume of the porous material used with the absorption rate for carbon dioxide and the volume of carbon dioxide absorbed of the carbon dioxide absorbent.

Examples 4 to 9 (Evaluation of Absorption Rate for Carbon Dioxide and Volume of Carbon Dioxide Absorbed of Carbon Dioxide Absorbent)

To the glass container with nitrogen replacement, 15 mg of the amine compound (A) described in Table 5, 10 g of methanol, and 1350 mg of the porous material (B) described in Table 5 were added and stirred for 10 hours to homogenize them. The resulting mixture was then placed under the circumstance of 40°C and 100 hPa, to distil the methanol off, followed by the vacuum drying at room temperature (23°C) for 24 hours to obtain the carbon dioxide absorbent.

(Measurement of Absorption Rate for Carbon Dioxide)

A petri dish with an inner diameter of 90 mm into which 500 mg of the carbon dioxide absorbent obtained was weighed, and a carbon dioxide concentration meter ("TR-76UI" produced by T & D Corporation) were set in a vacuum desiccator, which was then held for 1 hour in a thermo-hygrostat of 23°C and 50% RH, and the amount of change (ppm) in carbon dioxide concentration was calculated as the absorption rate for carbon dioxide (ppm/h). The results are shown in Table 5.

(Measurement of Volume of Carbon Dioxide Absorbed)

15 mg of the carbon dioxide absorbent obtained was set in a differential thermogravimetric analyzer ("EXSTER TGD6200" produced by Hitachi High-Tech Corporation) and stood at 45°C under a dry air environment for 6 hours, and an increased amount in mass of the solid carbon dioxide absorbent was measured. Here, as a gas used at measurements, air (flow rate: 200 ml/min) was used at absorbing carbon dioxide. The increased amount in the mass of the carbon dioxide absorbent was used as an amount of carbon dioxide (mg) absorbed, and the amount of carbon dioxide absorbed per 1 g of the carbon dioxide absorbent (mg/g) is shown as the volume of carbon dioxide absorbed in Table 5.

Examples 10 to 15

The carbon dioxide absorbent was prepared and evaluated by the same method as in Examples 4 to 9 except that in Examples 4 to 9, the amine compound (A) and the porous material (B) described in Table 6 were used; the amount of the amine compound (A) used was changed to 330 mg; and the amount of the porous material (B) used was changed to 1000 mg. The results are shown in Table 6.

Examples 16 to 21

The carbon dioxide absorbent was prepared and evaluated by the same method as in Examples 4 to 9 except that in Examples 4 to 9, the amine compound (A) and the porous material (B) described in Table 7 were used; the amount of the amine compound (A) used was changed to 600 mg; and the amount of the porous material (B) used was changed to 900 mg. The results are shown in Table 7.

The grades of the porous materials (B) used in Examples 4 to 21 are shown in Table 4.

[Table 4]

Table 4

| Grade of Porous Material (B) | $D_{50}$ (μm) | Specific Surface Area ($m^2/g$) | Pore Size (nm) | Pore Volume ($cm^3/g$) |
|---|---|---|---|---|
| Q3 | 75 | 739.2 | 2.3 | 0.40 |
| Q6 | 85 | 568.1 | 5.8 | 0.83 |
| Q10 | 100 | 309.5 | 16.6 | 1.11 |
| Q15 | 120 | 227.9 | 22.7 | 1.29 |
| Q30 | 125 | 108.7 | 47.6 | 1.29 |
| Q50 | 130 | 74.2 | 54.2 | 1.31 |

[Table 5]

Table 5

| Example | Amine Compound (A) | Porous Material (B) | (A) Content in $CO_2$ Absorbent (% by mass) | Specific Surface Area of $CO_2$ Absorbent ($m^2/g$) | Pore Size of $CO_2$ Absorbent (nm) | Pore Volume of $CO_2$ Absorbent ($cm^3/g$) | Burial Rate for Pore of $CO_2$ Absorbent (%) | $CO_2$ Absorption Rate (ppm/h) | Volume of $CO_2$ absorbed (mg/g) |
|---|---|---|---|---|---|---|---|---|---|
| 4 | P-MIBPA | Q3 | 10 | 294.5 | 2.9 | 0.212 | 47.00 | 74 | 11.6 |
| 5 | P-MIBPA | Q6 | 10 | 362.1 | 6.7 | 0.61 | 26.67 | 84 | 16.4 |
| 6 | P-MIBPA | Q10 | 10 | 216.7 | 18.3 | 0.89 | 19.55 | 117 | 17.6 |
| 7 | P-MIBPA | Q15 | 10 | 170.5 | 24.2 | 1.03 | 20.48 | 122 | 20.2 |
| 8 | P-MIBPA | Q30 | 10 | 87.3 | 33.9 | 1.02 | 21.05 | 132 | 20.8 |
| 9 | P-MIBPA | Q50 | 10 | 55.6 | 64.6 | 1.05 | 19.85 | 139 | 22.1 |

[Table 6]

Table 6

| Example | Amine Compound (A) | Porous Material (B) | (A) Content in $CO_2$ Absorbent (% by mass) | Specific Surface Area of $CO_2$ Absorbent ($m^2/g$) | Pore Size of $CO_2$ Absorbent (nm) | Pore Volume of $CO_2$ Absorbent ($cm^3/g$) | Burial Rate for Pore of $CO_2$ Absorbent (%) | $CO_2$ Absorption Rate (ppm/h) | Volume of $CO_2$ absorbed (mg/g) |
|---|---|---|---|---|---|---|---|---|---|
| 10 | P-MIBPA | Q3 | 25 | 8.0 | 5.2 | 0.010 | 97.50 | 88 | 27.3 |
| 11 | P-MIBPA | Q6 | 25 | 193.6 | 6.9 | 0.33 | 59.76 | 146 | 41.2 |
| 12 | P-MIBPA | Q10 | 25 | 171.4 | 18.7 | 0.70 | 36.67 | 156 | 47.3 |
| 13 | P-MIBPA | Q15 | 25 | 142.2 | 24.5 | 0.87 | 32.69 | 157 | 48.7 |
| 14 | P-MIBPA | Q30 | 25 | 67.0 | 52.9 | 0.89 | 31.42 | 162 | 51.7 |
| 15 | P-MIBPA | Q50 | 25 | 47.4 | 70.2 | 0.93 | 29.01 | 156 | 53.5 |

[Table 7]

Table 7

| Example | Amine Compound (A) | Porous Material (B) | (A) Content in $CO_2$ Absorbent (% by mass) | Specific Surface Area of $CO_2$ Absorbent ($m^2/g$) | Pore Size of $CO_2$ Absorbent (nm) | Pore Volume of $CO_2$ Absorbent ($cm^3/g$) | Burial Rate for Pore of $CO_2$ Absorbent (%) | $CO_2$ Absorption Rate (ppm/h) | Volume of $CO_2$ absorbed (mg/g) |
|---|---|---|---|---|---|---|---|---|---|
| 16 | P-MIBPA | Q3 | 40 | 1.9 | 6.8 | 0.003 | 99.25 | 19 | 38.7 |
| 17 | P-MIBPA | Q6 | 40 | 46.7 | 7.6 | 0.09 | 89.21 | 137 | 51.4 |
| 18 | P-MIBPA | Q10 | 40 | 97.8 | 21 | 0.40 | 63.78 | 162 | 67.0 |
| 19 | P-MIBPA | Q15 | 40 | 73.8 | 25.8 | 0.48 | 63.29 | 163 | 68.2 |
| 20 | P-MIBPA | Q30 | 40 | 40.3 | 54.9 | 0.55 | 57.12 | 169 | 67.6 |
| 21 | P-MIBPA | Q50 | 40 | 36.1 | 76.6 | 0.69 | 47.33 | 160 | 68.1 |

In Tables 5 to 7, as the amine compound and the porous materials, the following were used

(Amine Compound)

P-MIBPA: the amine compound obtained in Synthesis Example 1

(porous material)

Q3: porous silica particles "CARiACT Q3" (produced by Fuji Silysia Chemical Ltd.)

Q6: porous silica particles "CARiACT Q6" (produced by Fuji Silysia Chemical Ltd.)

Q10: porous silica particles "CARiACT Q10" (produced by Fuji Silysia Chemical Ltd.)

Q15: porous silica particles "CARiACT Q15" (produced by Fuji Silysia Chemical Ltd.)

Q30: porous silica particles "CARiACT Q30" (produced by Fuji Silysia Chemical Ltd.)

Q50: porous silica particles "CARiACT Q50" (produced by Fuji Silysia Chemical Ltd.)

From Tables 5 to 7, it is evident that when the pore size of the porous material (B) used is 5 nm or more, preferably 10 nm or more, the absorption rate for carbon dioxide and the volume of carbon dioxide absorbed are better, irrespective of the content of the amine compound (A). This is presumably because, when the pore size of the porous material (B) used is 5 nm or more, preferably 10 nm or more, a phenomenon can be circumvented in which the supported amine compound (A) blocks pores of the porous material (B) and interferes with flow paths for carbon dioxide. From this viewpoint, the burial rate for pores of the carbon dioxide absorbent is preferably 90% or less.
Likewise, it is evident that when the pore volume of the porous material (B) used is 0.5 $cm^3/g$ or more, preferably 0.7 $cm^3/g$ or more, more preferably 0.8 $cm^3/g$ or more, and even more preferably 1.0 $cm^3/g$ or more, the absorption rate for carbon dioxide and the volume of carbon dioxide absorbed are better.

Industrial Applicability

According to the present invention, the carbon dioxide absorbent with a good carbon dioxide absorption property and particularly excellent repeated usability, the method for capturing carbon dioxide using the carbon dioxide absorbent, and the carbon dioxide separation and capture apparatus can be provided.

Reference Signs List

| | |
|---|---|
| 100 | carbon dioxide separation and capture apparatus |
| 1 | absorption apparatus |
| 2 | desorption apparatus |
| 3 | connecting part |
| 11, 21 | reaction column |
| 12, 22 | absorbent holding part |
| 12a | carbon dioxide absorbent |
| 13 | gas supplying part |
| 21 | reaction column |
| 22a | carbon dioxide absorbent with carbon dioxide absorbed |
| 23 | gas discharging part |
| 24 | absorbent discharging part |

## Claims

**1.** A carbon dioxide absorbent comprising an amine compound (A) represented by the following general formula (1) and a porous material (B):

[Formula 1]

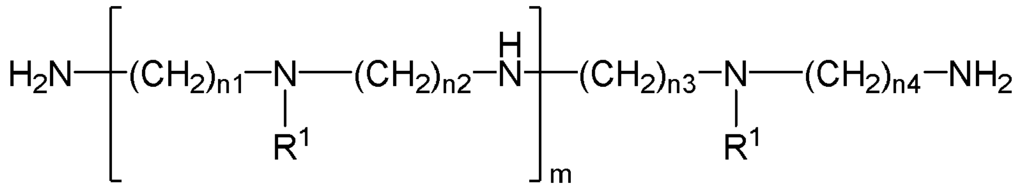

(1)

wherein in the formula (1), each $R^1$ independently represents a hydroxy group or an organic group having 1 to 10 carbon atoms; n1 to n4 each independently represent a number of 1 to 8; and m represents a number of 0 to 10.

**2.** The carbon dioxide absorbent according to claim 1, wherein in the general formula (1), n1 to n4 are each independently a number of 3 to 8, and m is a number of 1 to 10.

**3.** The carbon dioxide absorbent according to claim 1 or 2, wherein in the general formula (1), all of n1 to n4 are 3.

**4.** The carbon dioxide absorbent according to any one of claims 1 to 3, wherein at least some of the amine compound (A) is supported on the porous material (B).

**5.** The carbon dioxide absorbent according to any one of claims 1 to 4, wherein the porous material (B) comprises at least one selected from the group consisting of porous silica and porous alumina.

**6.** The carbon dioxide absorbent according to any one of claims 1 to 5, wherein the porous material (B) is in a particulate form.

**7.** The carbon dioxide absorbent according to claim 6, wherein a volume median particle size ($D_{50}$) of the porous material (B) as measured by laser diffraction/scattering particle size distribution measurement is 1 $\mu$m or more and 500 $\mu$m or less.

**8.** The carbon dioxide absorbent according to any one of claims 1 to 7, wherein a specific surface area of the porous material (B) as measured by a BET method is 2 $m^2$/g or more and 3,000 $m^2$/g or less.

**9.** The carbon dioxide absorbent according to any one of claims 1 to 8, wherein a pore size of the porous material (B) is 5 nm or more.

**10.** The carbon dioxide absorbent according to any one of claims 1 to 9, wherein a pore volume of the porous material (B) is 0.1 $cm^3$/g or more and 5.0 $cm^3$/g or less.

**11.** The carbon dioxide absorbent according to any one of claims 1 to 10, wherein a content of the amine compound (A) in the carbon dioxide absorbent is 0.1 parts by mass or more and 1,000 parts by mass or less with respect to 100 parts by mass of the porous material (B).

**12.** The carbon dioxide absorbent according to any one of claims 4 to 11, wherein a burial rate for pores of the carbon dioxide absorbent calculated according to the following expression is 90% or less:

Burial rate for pores (%) of the carbon dioxide absorbent = {Pore volume ($cm^3$/g) of the porous material (B) - Pore volume ($cm^3$/g) of the carbon dioxide absorbent} / Pore volume ($cm^3$/g) of the porous material (B) $\times$ 100.

**13.** A method for capturing carbon dioxide, comprising using the carbon dioxide absorbent according to any one of claims 1 to 12.

**14.** The method according to claim 13, wherein the method comprises:

an absorption step of bringing a gas containing carbon dioxide into contact with the carbon dioxide absorbent, to cause the carbon dioxide absorbent to absorb the carbon dioxide, and

a desorption step of desorbing carbon dioxide from the carbon dioxide absorbent with carbon dioxide absorbed in the absorption step, and
wherein the desorption step comprises at least one step selected from the group consisting of the following (I) to (III):

(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and
(III) a step of heating the carbon dioxide absorbent with carbon dioxide absorbed.

**15.** A carbon dioxide separation and capture apparatus comprising: an absorption apparatus comprising a mechanism for bringing a gas containing carbon dioxide into contact with the carbon dioxide absorbent according to any one of claims 1 to 12, to cause the carbon dioxide absorbent to absorb the carbon dioxide; and a desorption apparatus comprising a mechanism for desorbing carbon dioxide from the carbon dioxide absorbent with carbon dioxide absorbed.

[Fig. 1]

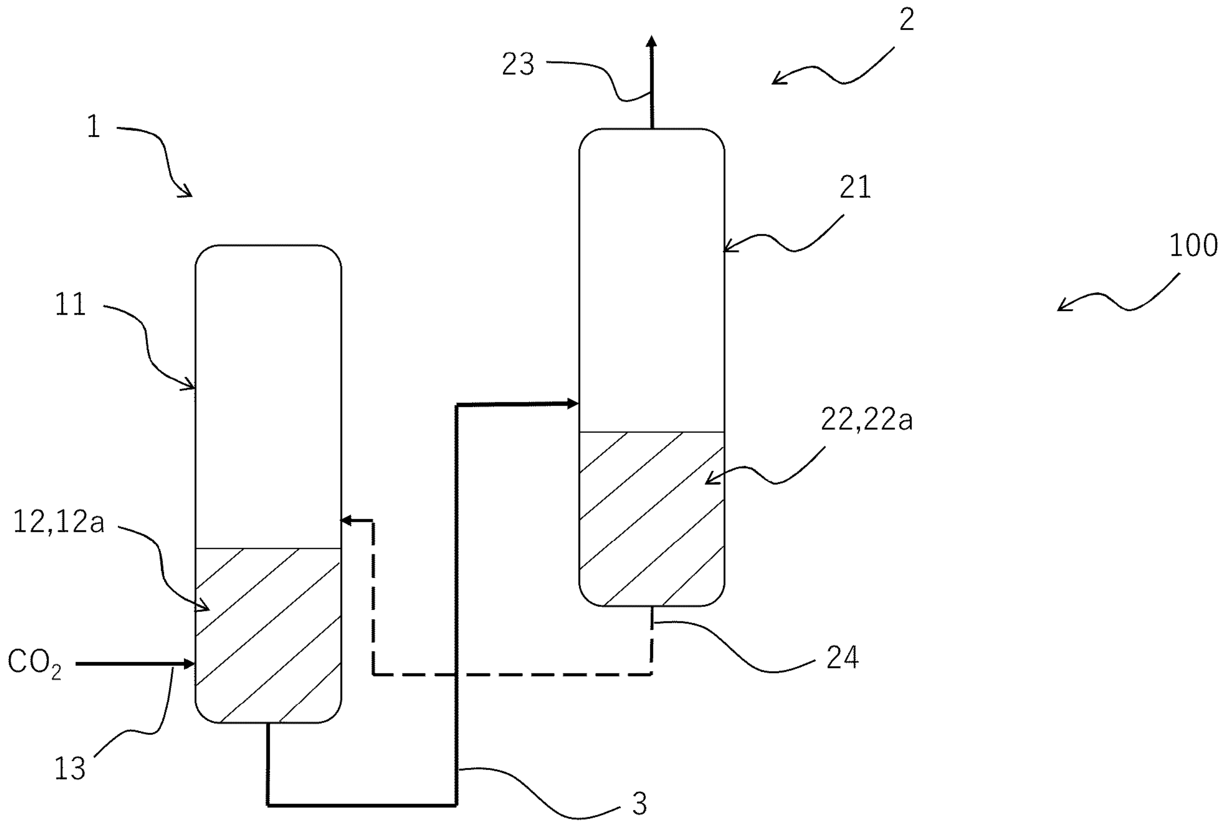

**INTERNATIONAL SEARCH REPORT**

International application No. **PCT/JP2024/015054**

**A. CLASSIFICATION OF SUBJECT MATTER**

***B01J 20/22***(2006.01)i; ***B01D 53/14***(2006.01)i; ***B01D 53/62***(2006.01)i; ***B01D 53/81***(2006.01)i; ***B01J 20/24***(2006.01)i; ***B01J 20/26***(2006.01)i; ***B01J 20/28***(2006.01)i; ***B01J 20/34***(2006.01)i; ***C01B 32/50***(2017.01)i; ***C07B 61/00***(2006.01)i; ***C07C 211/14***(2006.01)i

FI: B01J20/22 A; B01D53/14 100; B01D53/81; B01J20/34 E; B01J20/34 F; B01J20/34 H; C07C211/14; C01B32/50; C07B61/00 300; B01J20/26 A; B01J20/28 Z; B01D53/62 ZAB; B01J20/24 A

According to International Patent Classification (IPC) or to both national classification and IPC

**B. FIELDS SEARCHED**

Minimum documentation searched (classification system followed by classification symbols)

B01J20/22; B01D53/14; B01D53/62; B01D53/81; B01J20/24; B01J20/26; B01J20/28; B01J20/34; C01B32/50; C07B61/00; C07C211/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br><br>A | JP 2015-9185 A (RESEARCH INSTITUTE OF INNOVATIVE TECHNOLOGY FOR THE EARTH) 19 January 2015 (2015-01-19)<br>claims, paragraphs [0010], [0011], [0038]-[0064], examples, in particular, example 1 | 1-6, 8-11, 13-15<br><br>7, 12 |
| A | JP 2020-168624 A (KABUSHIKI KAISHA TOYOTA CHUO KENKYUSHO) 15 October 2020 (2020-10-15) | 1-15 |
| A | JP 2021-113182 A (TOSOH CORPORATION) 05 August 2021 (2021-08-05) | 1-15 |
| A | JP 61-71819 A (EXXON RESEARCH ENGINEERING CO.) 12 April 1986 (1986-04-12) | 1-15 |
| A | JP 2013-501608 A (SHELL INTERNATIONALE RESEARCH MAATSCHAPPIJ BESLOTEN VENNOOTSHAP) 17 January 2013 (2013-01-17) | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

INTERNATIONAL SEARCH REPORT

International application No.
**PCT/JP2024/015054**

C. DOCUMENTS CONSIDERED TO BE RELEVANT

| Category* | Citation of document, with indication, where appropriate, of the relevant passages | Relevant to claim No. |
|---|---|---|
| A | JP 2011-525423 A (BASF SE) 22 September 2011 (2011-09-22) | 1-15 |
| A | JP 2011-525422 A (BASF SE) 22 September 2011 (2011-09-22) | 1-15 |
| A | JP 2-504367 A (SOCIETE NATIONALE ELF AQUITAINE) 13 December 1990 (1990-12-13) | 1-15 |
| A | JP 7-31830 A (KYOWA HAKKO KOGYO CO., LTD.) 03 February 1995 (1995-02-03) | 1-15 |
| A | JP 4-29721 A (SUMITOMO SEIKA CHEMICALS CO., LTD.) 31 January 1992 (1992-01-31) | 1-15 |
| A | WO 2020/95934 A1 (KAWASAKI JUKOGYO KABUSHIKI KAISHA) 14 May 2020 (2020-05-14) | 1-15 |
| A | US 2019/0329176 A1 (THE BOARD OF TRUSTEES OF THE UNIVERSITY OF ILLINOIS) 31 October 2019 (2019-10-31) | 1-15 |
| A | US 2017/0246587 A1 (UNIVERSITY-INDUSTRY COOPERATION GROUP OF KYUNG HEE UNIVERSITY) 31 August 2017 (2017-08-31) | 1-15 |
| A | EP 2759328 A1 (IFP ENERGIES NOUVELLES) 30 July 2014 (2014-07-30) | 1-15 |
| A | US 2021/0039040 A1 (SOGANG UNIVERSITY RESEARCH FOUNDATION) 11 February 2021 (2021-02-11) | 1-15 |
| A | US 2011/0256043 A1 (BLAIR, Alan M.) 20 August 2011 (2011-08-20) | 1-15 |
| A | WO 2016/167258 A1 (OSAKA GAS CHEMICALS CO., LTD.) 20 October 2016 (2016-10-20) | 1-15 |
| P, A | WO 2023/181676 A1 (RESEARCH INSTITUTE OF INNOVATIVE TECHNOLOGY FOR THE EARTH) 28 September 2023 (2023-09-28) | 1-15 |
| E, A | JP 2024-59402 A (RESEARCH INSTITUTE OF INNOVATIVE TECHNOLOGY FOR THE EARTH) 01 May 2024 (2024-05-01) | 1-15 |

Form PCT/ISA/210 (second sheet) (July 2022)

**INTERNATIONAL SEARCH REPORT**
**Information on patent family members**

International application No.
**PCT/JP2024/015054**

| Patent document cited in search report | Publication date (day/month/year) | Patent family member(s) | Publication date (day/month/year) |
|---|---|---|---|
| JP 2015-9185 A | 19 January 2015 | US 2016/0144340 A1<br>claims, paragraphs [0014], [0015], [0044]-[0070], examples, in particular, example 1<br>WO 2014/208712 A1 | |
| JP 2020-168624 A | 15 October 2020 | (Family: none) | |
| JP 2021-113182 A | 05 August 2021 | (Family: none) | |
| JP 61-71819 A | 12 April 1986 | JP 1-159021 A<br>JP 52-5679 A<br>US 4112050 A<br>US 4112051 A<br>US 4112052 A<br>US 4217237 A<br>GB 1567943 A<br>GB 1567944 A<br>GB 1567945 A<br>DE 2628376 A1<br>FR 2316999 A1 | |
| JP 2013-501608 A | 17 January 2013 | US 2012/0216678 A1<br>WO 2011/018479 A1<br>CA 2769617 A1<br>CN 102470316 A | |
| JP 2011-525423 A | 22 September 2011 | US 2011/0094381 A1<br>WO 2009/156273 A1<br>CA 2727057 A1 | |
| JP 2011-525422 A | 22 September 2011 | US 2011/0135549 A1<br>WO 2009/156271 A1<br>CA 2726922 A1 | |
| JP 2-504367 A | 13 December 1990 | US 5277885 A<br>US 5348714 A<br>US 5209914 A<br>US 5366709 A<br>WO 1989/011327 A1<br>EP 348251 A1<br>FR 2631852 A1<br>FR 2631853 A1<br>FR 2640157 A1<br>DD 283777 A5 | |
| JP 7-31830 A | 03 February 1995 | (Family: none) | |
| JP 4-29721 A | 31 January 1992 | (Family: none) | |
| WO 2020/95934 A1 | 14 May 2020 | US 2021/0268428 A1<br>CN 111801151 A | |
| US 2019/0329176 A1 | 31 October 2019 | US 2022/0387926 A1 | |
| US 2017/0246587 A1 | 31 August 2017 | WO 2016/060508 A2<br>KR 10-1549950 B | |
| EP 2759328 A1 | 30 July 2014 | FR 2999448 A1 | |
| US 2021/0039040 A1 | 11 February 2021 | WO 2019/164081 A1<br>KR 10-2019-0101052 A | |

Form PCT/ISA/210 (patent family annex) (July 2022)

**INTERNATIONAL SEARCH REPORT**
**Information on patent family members**

International application No.
**PCT/JP2024/015054**

| Patent document cited in search report | Publication date (day/month/year) | Patent family member(s) | Publication date (day/month/year) |
|---|---|---|---|
| US 2011/0256043 A1 | 20 August 2011 | US 2014/0138578 A1 | |
| WO 2016/167258 A1 | 20 October 2016 | (Family: none) | |
| WO 2023/181676 A1 | 28 September 2023 | (Family: none) | |
| JP 2024-59402 A | 01 May 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012139622 A
- JP 2018187574 A
- JP 2020058966 A